**Europäisches Patentamt**

(19) **European Patent Office**

**Office européen des brevets**

(11) Veröffentlichungsnummer: **0 036 040**

**A1**

(12) **EUROPÄISCHE PATENTANMELDUNG**

(21) Anmeldenummer: **80107652.2**

(22) Anmeldetag: **05.12.80**

(51) Int. Cl.³: **A 61 N 5/04**

(30) Priorität: **18.03.80 DE 3010339**

(43) Veröffentlichungstag der Anmeldung:
**23.09.81 Patentblatt 81/38**

(84) Benannte Vertragsstaaten:
**AT BE CH DE FR GB IT LI LU NL SE**

(71) Anmelder: **ROBERT BOSCH GMBH**
**Postfach 50**
**D-7000 Stuttgart 1(DE)**

(72) Erfinder: **Walter, John, Prof.Dr.-Ing.**
**Hans-Böhm-Zeile 33 a**
**D-1000 Berlin 37(DE)**

(74) Vertreter: **Schmidt, Hans-Ekhardt**
**Robert Bosch GmbH Geschäftsbereich Elektronik**
**Patent- und Lizenzabteilung Forckenbeckstrasse 9-13**
**D-1000 Berlin 33(DE)**

(54) **Mikrowellen-Kontaktstrahler für Diathermieanwendungen.**

(57) Es wird ein Mikrowellen-Kontaktstrahler für Diathermie-anwendungen vorgeschlagen, der als Hohlleiteranordnung, vorzugsweise als Rundhohlleiter, ausgebildet ist. In dieser Hohlleiteranordnung werden zirkular polarisierte Wellen erzeugt, wobei die Hohlleiteranordnung eine besonders geringe Streustrahlung und die Zirkularpolarisation eine besonders homogene Erwärmung des aufliegenden Gewe-bes bewirkt. Um für örtlich begrenzte Anwendungen Strahler mit geringen Abmessungen zu erhalten, wird weiter vorge-schlagen, in die Hohlleiteranordnung leitende Stege einzu-bringen, wodurch die Grenzfrequenz vermindert wird.

Fig. 1

26/80
EK/PLI Wt/Li
17. 3.  1980


ROBERT BOSCH GMBH, 7000 Stuttgart 1 .


Mikrowellen-Kontaktstrahler
für Diathermieanwendungen


Stand der Technik


Die Erfindung geht aus von einem Mikrowellen-Kontaktstrahler
nach der Gattung des Hauptanspruchs.

Es ist bekannt, für Diathermieanwendungen Mikrowellen-
Kontaktstrahler zu verwenden. Dabei werden Mikrowellen, üblicherweise im Frequenzbereich um 2 GHz, erzeugt und über
einen Strahler auf das zu erwärmende Gewebe aufgestrahlt.
Die bekannten Strahler sind dabei in der Regel als Hohlspiegelantennen ausgebildet, wobei die Mikrowelle über eine induktive
Schleifenanordnung in die Antenne eingekoppelt wird. Diese
Strahleranordnungen haben jedoch den Nachteil, daß aufgrund
der relativ ungerichteten Abstrahlung eine Streustrahlung auftritt, die zu einer Belastung benachbarter Gewebegebiete und
des Bedienungspersonals führt. Dies ist insbesondere bei Kopfbestrahlungen von Nachteil, bei denen man nur ein bestimmtes,
räumlich eng begrenztes Gewebegebiet möglichst homogen erwärmen
will.

Außerdem haben die üblicherweise verwendeten linear polarisierten Mikrowellen den Nachteil, daß eine Wärmeentwicklung im Gewebe richtungsabhängig wird, weil die Wärmeentwicklung davon abhängt, ob die Feldlinien parallel oder nichtparallel zu Gefäßen und anderen Inhomogenitäten im Gewebe verlaufen.

Vorteile der Erfindung

Der erfindungsgemäße Mikrowellen-Kontaktstrahler mit den kennzeichnenden Merkmalen des Hauptanspruchs hat demgegenüber den Vorteil, durch Verwendung einer Hohlleiteranordnung als Kontaktstrahler nur eine äußerst geringe Streustrahlung zu erzeugen.

Durch die in den Unteransprüchen aufgeführten Maßnahmen sind vorteilhafte Weiterbildungen des im Hauptanspruch angegebenen Kontaktstrahlers möglich.

So wird in einer bevorzugten Ausführungsform der Erfindung eine zirkular polarisierte $H_{11}$-Welle in einem Rundhohlleiter erzeugt, der in seiner Auflagefläche mit einer Quarzscheibe abgeschlossen ist. Dabei bewirkt die zirkular polarisierte Welle im Gegensatz zur linear polarisierten nach dem Stande der Technik eine besonders homogene Erwärmung des Gewebegebietes, und die Quarzscheibe hat eine besonders gute Anpassung mit nur geringen Reflexionen zur Folge.

Die Streustrahlung wird erfindungsgemäß durch einen um die Auflagefläche herum angebrachten sogenannten $\lambda/4$-Choke weiter vermindert.

In einer weiteren, besonders bevorzugten Ausführungsform der Erfindung, insbesondere für Kopfbestrahlungen, werden die Abmessungen des Strahlers weiter dadurch vermindert, daß die Grenzfrequenz der Hohlleiteranordnung durch leitende Stege
vermindert wird. Hierdurch ist beispielsweise der Aufbau
einer Rundhohlleiteranordnung für die üblichen Betriebsfrequenzen mit nur sehr geringem Durchmesser der Auflagefläche
möglich.

Zeichnung

Die Erfindung ist in der Zeichnung dargestellt und in der
nachfolgenden Beschreibung näher erläutert. Es zeigen:

Fig. 1   einen Längsschnitt durch eine Rundhohlleiteranord-
         nung in der Antennenebene;

Fig. 2   einen Längsschnitt durch die Rundhohlleiteranord-
         nung in der Polarisatorebene;

Fig. 3   einen Querschnitt durch die Rundhohlleiteranordnung
         in der Antennenebene;

Fig. 4   einen Querschnitt durch die Rundhohlleiteranordnung
         in der Polarisatorebene;

Fig. 5   einen Längsschnitt durch eine zweite Rundhohlleiteranordnung mit vermindertem Durchmesser;

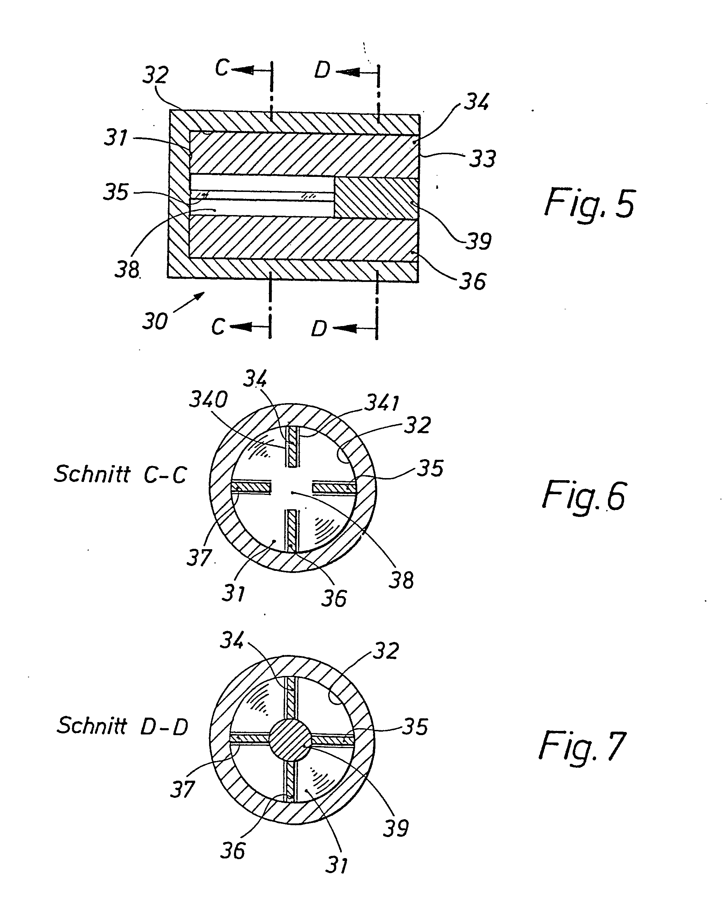

Fig. 6
und  7   zwei Querschnitte durch die in Fig. 5 dargestellte
         zweite Rundhohlleiteranordnung.

Beschreibung der Ausführungsbeispiele

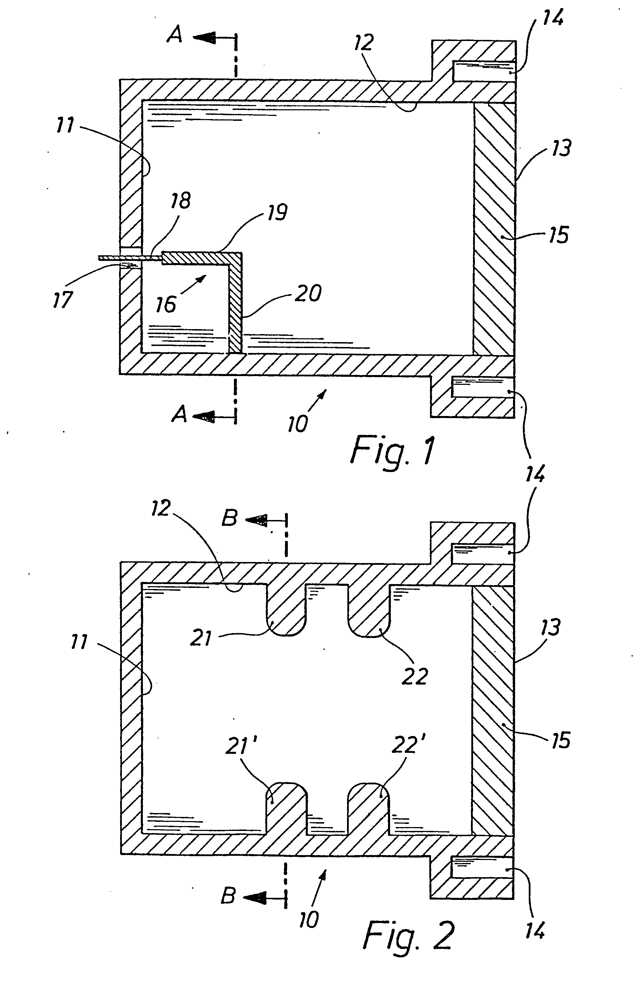

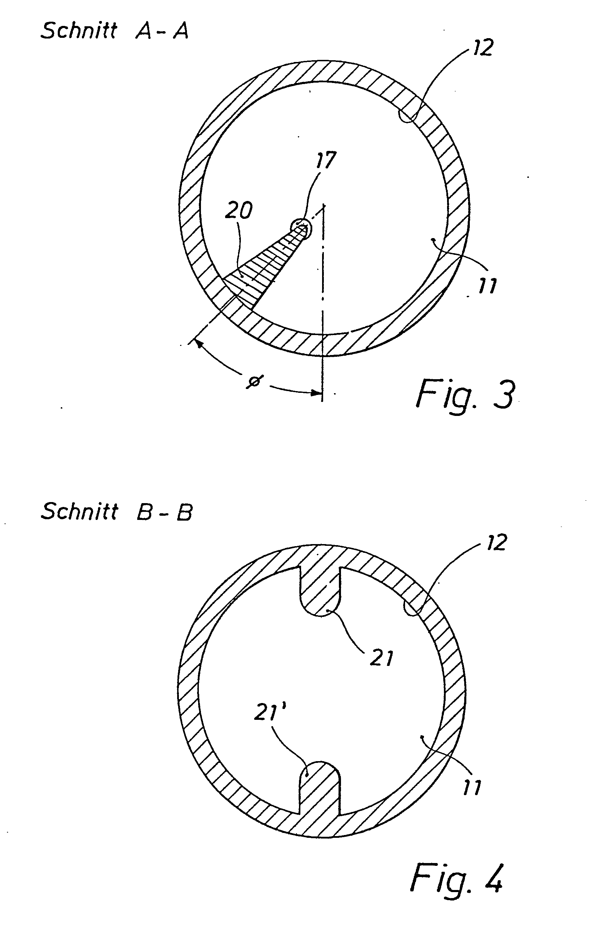

In den Fig. 1 bis 4 ist ein erstes Ausführungsbeispiel eines
erfindungsgemäßen Mikrowellen-Kontaktstrahlers dargestellt.
Dabei ist in den Fig. 1 und 2 mit 10 eine Rundhohlleiteranordnung bezeichnet, wobei die Längsschnitte der Fig. 1 und 2 um
einen Winkel $\phi$ gegeneinander versetzt sind, wie dies aus den in
den Fig. 3 und 4 dargestellten Querschnitten ersichtlich ist.

Die Rundhohlleiteranordnung 10 ist an ihrem einen Ende durch
die Rückwand 11 abgeschlossen, das andere Ende bildet die
Auflagefläche 13; in radialer Richtung wird sie durch die
Innenwand 12 begrenzt. Bei dem dargestellten ersten Ausführungsbeispiel ist dabei rings um die Auflagefläche 13 herum
eine Nut 14 angeordnet, die als $\lambda/4$-Choke dient und eine
weitere Herabsetzung der Streustrahlung der Rundhohlleiteranordnung 10 bewirkt. Der Hohlleiter ist in seiner Auflagefläche 13 mit einer Quarzscheibe 15 abgeschlossen, die als
$\lambda/4$-Transformator dient und eine Herabsetzung der Fehlanpassung zwischen Strahler und Gewebe bewirkt. In der Rundhohlleiteranordnung 10 wird eine $H_{11}$-Welle erzeugt, und zwar über
eine induktive Schleife 16, die vom Außenraum durch eine Öffnung 17 in der Rückwand 11 hindurch axial geführt ist und
sich nach einer vorgegebenen Länge radial zur Innenwand 12
hin erstreckt. Es versteht sich jedoch von selbst, daß auch
kapazitive Anregung mit einem radial angeordneten Stift möglich ist. Wie aus den Fig. 1 und 3 ersichtlich, hat der
axiale Teil dabei einen kreisförmigen und der radiale einen
rechteckförmigen Querschnitt. Der axiale Teil der Schleife 16
unterteilt sich in zwei Abschnitte 18 und 19, von denen der
erste Abschnitt 18 sich durch die Öffnung 17 hindurch bis in
den Innenraum der Hohlleiteranordnung 10 erstreckt und danach
in den zweiten Abschnitt 19 übergeht. Der erste Abschnitt 18
hat dabei einen geringeren Durchmesser als der zweite
Abschnitt 19. Dies dient dazu, um den Widerstand des durch
die Abschnitte 18, 19 gebildeten Teils der Schleife 16 herabzusetzen. Der dritte Abschnitt 20 der Schleife 16 bildet den
eigentlichen Anregungsteil der Schleife 16. Wie bereits
erwähnt, ist sein Querschnitt rechteckig, wobei die radiale
Seite des Rechtecks zur Innenwand 12 hin größer wird. Diese
Formgebung gestattet es, das Ankopplungsverhalten der
Schleife 16 in der Rundhohlleiteranordnung 10 genau vorauszuberechnen.

Wie aus den Fig. 2 und 4 ersichtlich, erstrecken sich in den
Innenraum der Rundhohlleiteranordnung hinein von der Innenwand 12 aus zapfenförmige Polarisatoren 21, 21'; 22, 22',
wobei sich jeweils zwei Polarisatoren paarweise gegenüberstehen. Die durch diese Polarisatorpaare 21, 21' gebildete
Achse ist gegen die vom dritten Abschnitt 20 der Schleife 16
gebildete Achse um einen Winkel $\phi$ versetzt, wie ein Vergleich
der Fig. 3 und 4 zeigt. Hierdurch ist es möglich, eine
Zirkularpolarisation mit besonders geringer Restelliptizität
zu erzeugen.

In den Fig. 5 bis 7 ist eine zweite Ausführungsform eines
erfindungsgemäßen Mikrowellen-Kontaktstrahlers dargestellt.
Wie bei dem ersten Ausführungsbeispiel wird dabei eine Rundhohlleiteranordnung 30 verwendet, die von einer Rückwand 31,
einer Innenwand 32 und einer Auflagefläche 33 begrenzt ist,
wie dies die Fig. 5 bis 7 zeigen.

Die für manche Anwendungen gewünschten kleinen Abmessungen
eines Kontaktstrahlers können für eine gegebene Betriebsfrequenz nicht immer realisiert werden, weil die Abmessungen
einer Rundhohlleiteranordnung durch die Grenzfrequenz dieser
Anordnung bestimmt ist. Will man also die Abmessungen, insbesondere den Durchmesser der Rundhohlleiteranordnung verringern,
ist es erforderlich, durch zusätzliche Maßnahmen eine Verringerung der Grenzfrequenz herbeizuführen. Erfindungsgemäß werden hierzu bei dem in den Fig. 5 bis 7 dargestellten zweiten
Ausführungsbeispiel der Erfindung leitende Stege 34, 35, 36, 37
vorgesehen, die in axialer Richtung an der Innenwand 32 verlaufen. In besonders vorteilhafter Weise können diese leitenden
Stege durch kaschierte Leiterplatten realisiert werden. Hierzu
werden beispielsweise zwei mit entsprechenden Ausbrüchen versehene kaschierte Leiterplatten unter 90$^{\circ}$ ineinandergesteckt
und in die Hohlleiteranordnung eingeführt. Nach Verbindung der
leitenden Stege 34 bis 37 mit der Innenwand 32 wird alsdann der innere Teil der Leiterplatten herausgeknackt, so daß ein freier Innenraum 38 entsteht, wie dies insbesondere aus Fig. 6 ersichtlich ist. Die beispielsweise auf diese Art hergestellten leitenden Stege 34 bis 37 bewirken nun eine Verminderung der Grenzfrequenz der Rundhohlleiteranordnung 30 in der Weise, daß bei Verwendung derselben Betriebsfrequenz wie für das erste Ausführungsbeispiel ein deutlich geringerer Durchmesser der Rundhohlleiteranordnung 30 erzielt wird. Zur Erzeugung einer zirkular polarisierten Welle bei dem zweiten Ausführungsbeispiel der Erfindung ist es erforderlich, in zwei Stegen, beispielsweise 34 und 35, eine jweils um 90° versetzte Mikrowelle anzuregen. Dies ist in vorteilhafter Weise dadurch möglich, daß von einer gemeinsamen Anschlußstelle zu den Stegen 34, 35 führende Anschlußleitungen einen Längenunterschied von $\lambda/4$ aufweisen.

Um eine möglichst reflexionsarme Anpassung an das Gewebe auch beim dargestellten zweiten Ausführungsbeispiel entsprechend den Fig. 5 bis 7 zu erzielen, ist schließlich ein dielektrischer Anpaßzylinder 39 vorgesehen, der von vorn in den Zwischenraum 38 eingeschoben wird, so daß er in der Auflagefläche 33 mit der Rundhohlleiteranordnung 30 abschließt.

Durch diese Maßnahmen wird weiterhin eine Feldkonzentration in Achsennähe bewirkt, so daß die Intensität der Strahlung in der Nähe der Berandung der Auflagefläche 33 abnimmt. Dies ist jedoch von besonderem Vorteil für solche Anwendungen, bei denen eine lokal strikt begrenzte Bestrahlung wünschenswert ist, beispielsweise bei der Bestrahlung von Nebenhöhlen, bei der auch bei kleinen Kontaktstrahlern die Berandung des Strahlers bis zum Auge reichen kann.

26/80
EK/PLI Wt/Li
17. 3.  1980

ROBERT BOSCH GMBH, 7000 Stuttgart 1

Ansprüche

1. Mikrowellen-Kontaktstrahler für Diathermieanwendungen, dadurch gekennzeichnet, daß eine Hohlleiteranordnung Anwendung findet, in der eine zirkular polarisierte Welle erzeugt und auf das aufliegende Gewebe aufgestrahlt wird.

2. Kontaktstrahler nach Anspruch 1, dadurch gekennzeichnet, daß eine Rundhohlleiteranordnung (10, 30) Verwendung findet, deren eines Ende mit einer Rückwand (11, 31) abgeschlossen ist und deren anderes Ende als Auflagefläche (13, 33) ausgebildet ist.

3. Kontaktstrahler nach Anspruch 1 oder 2, dadurch gekennzeichnet, daß eine $H_{11}$-Welle erzeugt wird.

4. Kontaktstrahler nach einem der vorhergehenden Ansprüche, dadurch gekennzeichnet, daß um die Auflagefläche (13, 33) herum eine als $\lambda/4$-Leitung wirkende Nut (14) angeordnet ist.

5. Kontaktstrahler nach einem der Ansprüche 3 oder 4, dadurch gekennzeichnet, daß zur Erzeugung der Zirkularpolarisation der Welle wenigstens ein Paar sich gegenüberstehender zapfenförmiger Polarisatoren (21, 21'; 22, 22') in den Innenraum der Hohlleiteranordnung ragt.

6. Kontaktstrahler nach Anspruch 5, dadurch gekennzeichnet, daß die Hohlleiteranordnung in der Auflagefläche (13) mit einer dielektrischen Scheibe, vorzugsweise einer Quarzscheibe (15), abgeschlossen ist.

7. Kontaktstrahler nach Anspruch 5 oder 6, dadurch gekennzeichnet, daß die Welle über eine induktiv wirkende
   Schleife (16) angeregt wird.

8. Kontaktstrahler nach Anspruch 7, dadurch gekennzeichnet,
   daß die Schleife (16) vom Außenraum durch eine Öffnung
   (17) in der Rückwand (11) der Hohlleiteranordnung hindurch axial geführt ist und sich nach einer vorgegebenen
   Länge radial zur Innenwand (12) hin erstreckt.

9. Kontaktstrahler nach Anspruch 8, dadurch gekennzeichnet,
   daß die Schleife (16) im axialen Bereich einen kreisförmigen Querschnitt hat, wobei sich der Durchmesser im
   Innenraum hinter der Öffnung (17) vergrößert.

10. Kontaktstrahler nach Anspruch 8 oder 9, dadurch gekennzeichnet, daß die Schleife (16) in einer axialen Ebene
    geführt ist, die gegenüber der von dem wenigstens einen
    Paar zapfenförmiger Polarisatoren (21, 21'; 22, 22') um
    einen vorgegebenen Winkel (φ) versetzt ist.

11. Kontaktstrahler nach einem der Ansprüche 1 bis 4,
    dadurch gekennzeichnet, daß zur Verminderung der Grenzfrequenz der Hohlleiteranordnung an der Innenwand (32)
    axial verlaufende Stege (34 bis 37) eingesetzt sind.

12. Kontaktstrahler nach Anspruch 11, dadurch gekennzeichnet,
    daß die Stege (34 bis 37) aus beschichteten Leiterplatten gebildet sind.

13. Kontaktstrahler nach Anspruch 11 oder 12, dadurch
    gekennzeichnet, daß die Erzeugung der Stegpaare (34, 36;
    35, 37) 90° phasenverschoben erfolgt.

26/80 - 3 -

14. Kontaktstrahler nach Anspruch 13, dadurch gekennzeichnet,
daß die Anregung der Welle an zwei benachbarten Stegen
(34 bis 37) erfolgt.

15. Kontaktstrahler nach einem der vorhergehenden Ansprüche,
dadurch gekennzeichnet, daß in den Zwischenraum zwischen
den Stegen (34 bis 37) axial ein dielektrischer Anpaßzylinder (39) eingesetzt ist, der mit der Auflagefläche
(33) abschließt.

16. Kontaktstrahler nach einem der vorhergehenden Ansprüche,
dadurch gekennzeichnet, daß die Hohlleiteranordnung aus
einem leitend beschichteten Kunststoff hergestellt ist.

1 / 3

Fig. 1

Fig. 2

213

Schnitt  A - A

*Fig. 3*

Schnitt  B - B

*Fig. 4*

Fig. 5

Schnitt C-C

Fig. 6

Schnitt D-D

Fig. 7

Europäisches
Patentamt

**EUROPÄISCHER RECHERCHENBERICHT**

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 61 N 5/04 |
| X | INSTITUTE OF ELECTRICAL AND ELECTRONIC ENGINEERS, International Microwave Symposium Digest, San Diego, 21-23. Juni 1977 The Institute of Electrical and Electronic Engineers, Inc. NEW YORK (US) G. KANTOR et al.:"The design and performance of a circularly polarized direct contact applicator for microwave diathermy" Seiten 364 bis 367 <br><br> * Seite 364, Abschnitt 2; Seite 365, erster Abschnitt * <br><br> -- | 1,2,4, 5,10 | |
| | US - A - 3 527 227 (FRITZ) <br><br> * Spalte 2, Zeilen 28-50; Spalte 3, Zeilen 4-6 und 39-50 * | 1,2,4, 15,16 | **RECHERCHIERTE SACHGEBIETE (Int. Cl.³)** <br><br> A 61 N 5/04 <br> H 05 B 6/72 <br> H 01 Q 13/08 <br> H 02 P 1/17 |
| | & DE - A - 1 539 955 <br><br> * Seite 2, Zeilen 13-20 * | 4 | |
| | & DE - A - 1 489 883 <br><br> * Seite 2, Zeilen 12,13 * <br><br> -- | 6 | |
| | DE - B - 1 006 086 (DEUTSCHE ELEKTRONIK) <br><br> * Spalte 1, Zeilen 29-37; Spalte 3, Zeilen 10-35 * <br><br> -- | 4,6,15 | **KATEGORIE DER GENANNTEN DOKUMENTE** <br><br> X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze |
| | GB - A - 835 944 (PHILIPS) <br><br> * Seite 1, Zeilen 67-86; Seite 2, Zeilen 35-41 * <br><br> -- ./. | 4,6,7 | E: kollidierende Anmeldung <br> D: in der Anmeldung angeführtes Dokument <br> L: aus andern Gründen angefuhrtes Dokument <br> &: Mitglied der gleichen Patent- familie, übereinstimmendes Dokument |

| | Der vorliegende Recherchenbericht wurde fur alle Patentansprüche erstellt. | | |
|---|---|---|---|
| Rechercheort <br> Den Haag | Abschlußdatum der Recherche <br> 23.06.1981 | Prufer <br> SIMON | |

EPA form 1503.1   06.78

**Europäisches Patentamt**

**EUROPÄISCHER RECHERCHENBERICHT**

| | EINSCHLÄGIGE DOKUMENTE | | | KLASSIFIKATION DER ANMELDUNG (Int.Cl. 3) |
|---|---|---|---|---|
| Kategorie | Kennzeichnung des Dokuments mit Angabe, soweit erforderlich, der maßgeblichen Teile | betrifft Anspruch | | |
| | FR – A – 2 118 859 (THOMSON-VARIAN) <br><br> * Seite 4, Zeilen 20-25 * <br><br> -- | 7-9 | | |
| | IRE TRANSACTIONS ON ANTENNAS AND PROPAGATION, Band AP-9, März 1961 NEW YORK (US) "Octave-Bandwidth Feed Horn for Paraboloid" Seiten 223 und 224 <br><br> * Seite 223, erster Abschnitt; Figur 1 * <br><br> -- | 3,11 | | RECHERCHIERTE SACHGEBIETE (Int. Cl. 3) |
| | US – A – 2 933 731 (FOSTER) <br><br> * Spalte 2, Zeile 66 bis Spalte 3, Zeile 19 * <br><br> -- | 11 | | |
| | US – A – 3 825 863 (MEIER) <br><br> * Spalte 1, Zeilen 30-38; Spalte 3, Zeile 51 bis Spalte 4, Zeile 9 * <br><br> ------ | 12 | | |